# EUROPÄISCHE PATENTANMELDUNG

(11) **EP 3 366 192 A1**
(43) Veröffentlichungstag der Anmeldung: **29.08.2018**
(21) Anmeldenummer: 18166579.5
(22) Anmeldetag: 22.09.2014
(51) Int. Cl.: A61B 1/00, A61B 90/00, G02B 23/24

(54) **VORRICHTUNG ZUM AUFNEHMEN EINES BILDES EINES OBJEKTFELDS AN EINEM MENSCHLICHEN ODER TIERISCHEN KÖRPER**

(30) Priorität: 24.09.2013 DE 102013110543
(62) Teilanmeldung aus: 14185795.3
(71) Anmelder: Karl Storz SE & Co. KG, 78532 Tuttlingen (DE)
(72) Erfinder: Heni, Andreas, 78532 Tuttlingen (DE); Kupferschmid, Markus, 78532 Tuttlingen (DE); Ulmschneider, Daniel, 78532 Tuttlingen (DE)

(57) **Zusammenfassung**

Eine erfindungsgemäße Vorrichtung zum Aufnehmen eines Bildes eines Objektfelds an einem menschlichen oder tierischen Körper von außerhalb des Körpers umfasst einen Schaft (2) und eine an einem distalen Ende des Schafts (2) angeordnete Beobachtungsoptik zum Aufnehmen des Bildes des Objektfelds, wobei die Beobachtungsoptik als Stereooptik mit mindestens einem elektronischen Bildaufnehmer zum Aufnehmen eines Stereobilds des Objektfelds ausgebildet ist und wobei die Vorrichtung eine Optikeinheit (20) aufweist, die die Beobachtungsoptik umfasst und die um eine zu einer Blickrichtung der Beobachtungsoptik näherungsweise parallele erste Drehachse drehbar ist.

## Beschreibung

Die vorliegende Erfindung betrifft eine Vorrichtung zum Aufnehmen eines Bildes eines Objektfelds an einem menschlichen oder tierischen Körper nach dem Oberbegriff von Anspruch 1.

Aus DE 10 2011 054 031 A1 ist eine Vorrichtung zum Beobachten und Beleuchten eines Objektfelds an einem Patienten von einer Stelle abseits des Körpers des Patienten bekannt, die eine Optik zum Beobachten des Objektfelds und eine Beleuchtung zum Beleuchten des Objektfelds aufweist. Die Vorrichtung weist weiterhin einen Schaft auf, an dessen distalem Ende ein gegenüber dem Schaft erweitertes Kopfteil angeordnet ist, in dem zumindest eine ausstrahlende Beleuchtungseinheit zur homogenen Ausleuchtung des Objektfelds angeordnet ist. Im Schaft verlaufen Zuleitungen für die zumindest eine Beleuchtungseinheit. Ferner kann der langerstreckte Schaft einen Bildweiterleiter aufnehmen, der das Bild des Operationsfelds zu einem proximalen Ende des Schafts weiterleitet. Eine derartige Vorrichtung wird auch als "Exoskop" bezeichnet. Exoskope der genannten Art werden von der Fa. KARL STORZ GmbH & Co. KG unter der Bezeichnung VITOM ® angeboten.

Ein solches Exoskop ermöglicht insbesondere die Beleuchtung und Beobachtung eines Operationsfelds bei einer chirurgischen Operation aus einem Arbeitsabstand von beispielsweise 20 bis 75 cm, so dass der Arbeitsraum des Operateurs durch das Exoskop praktisch nicht eingeschränkt wird. Der relativ schlanke Schaft nimmt die Leitungen zum Kopfteil auf, so dass keine freiliegenden Leitungen notwendig sind, die den Operateur behindern könnten. Durch Anschluss einer Videokamera wird es ermöglicht, das Bild des Objektfelds auf einem Bildschirm darzustellen, so dass dieses für den Operateur ermüdungsfrei beobachtbar ist; ferner wird hierdurch die Beobachtung des Objektfelds durch weitere Personen sowie die Aufzeichnung des Bilds beispielsweise für Dokumentationszwecke ermöglicht.

Das Exoskop kann durch eine Halterung gehalten werden, die einen gelenkigen Arm aufweist, an dessen distalem Ende ein Greifer angeordnet ist, der zum festen Halten des Exoskops, beispielsweise zum Greifen und Klemmen des Schafts ausgebildet ist. Durch Verstellen der Halterung kann das Exoskop je nach den gestellten Anforderungen in einem geeigneten Abstand zum Objektfeld und in einer geeigneten Position und Ausrichtung fixiert werden. Wenn die Optik des Exoskops als Seitblickoptik ausgebildet ist mit einer Blickrichtung, die mit der Längsachse des Schafts einen Winkel von etwa von 90° bildet, kann das Exoskop zum Beobachten eines horizontal angeordneten Objektfelds, das etwa ein Operationsfeld bei einer chirurgischen Operation am menschlichen Körper sein kann, insbesondere über dem Objektfeld mit senkrecht nach unten gerichteter Blickrichtung positioniert werden. Durch Verstellen der Halterung kann das Exoskop dann, je nach Bedarf und je nachdem von welcher Seite ein ungehinderter Zugang zum Operationsfeld erforderlich ist, bei näherungsweise gleichbleibender Blickrichtung in unterschiedliche Positionen gebracht und hierfür um eine vertikale Achse geschwenkt werden.

Bei dem bekannten Exoskop ist die Optik als monokulare Optik ausgebildet, wodurch nur eine begrenzte räumliche Wahrnehmung des Objektfelds möglich ist. Bei der Durchführung einer chirurgischen Operation kann jedoch eine räumliche Wahrnehmung des Objektfelds für den Operateur hilfreich sein. In anderen Anwendungsgebieten wie der Endoskopie ist bekannt, dass eine verbesserte räumliche Wahrnehmung des Objektfelds mit einer stereoskopischen Optik, bei der zwei Bilder des Objektfelds aus etwas unterschiedlicher Perspektive aufgenommen werden, möglich ist. Die beiden Bilder, die gemeinsam das stereoskopische Bild darstellen, werden auch als "Halbbilder" bezeichnet. Zur Aufnahme der beiden Halbbilder sind zwei Beobachtungsstränge erforderlich, die insbesondere jeweils ein Objektiv umfassen, wobei die Stereobasis durch den Abstand der beiden Objektive senkrecht zur Blickrichtung bestimmt wird. Die beiden Halbbilder werden einem Beobachter derart dargestellt, dass der Beobachter einen räumlichen Eindruck des Objektfelds gewinnt. Hierfür kann in an sich bekannter Weise beispielsweise ein Bildschirm mit einer wechselnden Polarisierung vorgesehen sein, wobei der Beobachter eine Polarisationsbrille mit unterschiedlichen Polarisierungen der beiden Gläser trägt.

Bei einer gattungsgemäßen Vorrichtung zum Aufnehmen eines Bildes eines Objektfelds an einem menschlichen oder tierischen Körpers stellt sich hierbei jedoch das Problem, dass sich bei einer Drehung des Schafts um eine näherungsweise parallel zur Blickrichtung der Optik stehende Achse das auf einem elektronischen Bildaufnehmer erzeugte Bild und somit auch das auf einem Bildschirm dargestellte Bild des Operationsfelds dreht. Bei Verwendung einer Stereooptik dreht sich zusätzlich die Basislinie der stereoskopischen Optik; die Stereobasis wird daher nicht mehr horizontal auf dem Bildschirm dargestellt, so dass der Stereoeffekt und damit auch der räumliche Eindruck verloren gehen können. Für einen Operateur, der den Bildschirm betrachtet, wird dadurch die Orientierung im Operationsfeld erschwert oder sogar unmöglich gemacht.

Aus JP 10192233 A ist ein stereoskopisches elektronisches Endoskop mit einer Schrägblickoptik bekannt, wobei eine motorische Verstellung eines Rotationselements vorgesehen ist, das ein optisches System mit zwei quer zur optischen Achse beabstandeten Objektivlinsensystemen und zwei diesen zugeordneten CCD-Bildaufnehmern trägt. In US 5,689,365 ist ein stereoskopisches Endoskop offenbart, das ein vorderes optisches System mit einer einzigen optischen Achse und ein rückwärtiges optisches System mit einer Mehrzahl optischer Achsen aufweist. Das rückwärtige optische System kann gemeinsam mit zwei photoelektrischen Bildaufnehmern relativ zum vorderen optischen System um eine zum Schaft des Endoskops parallele Achse gedreht werden.

Es ist Aufgabe der vorliegenden Erfindung, eine gattungsgemäße Vorrichtung anzugeben, die eine räumliche Darstellung bzw. Wahrnehmung des Objektfelds ermöglicht, wobei die genannten Nachteile vermieden werden können.

Diese Aufgabe wird durch eine Vorrichtung gemäß Anspruch 1 gelöst. Vorteilhafte Weiterbildungen der Erfindung ergeben sich aus den Unteransprüchen.

Eine erfindungsgemäße Vorrichtung zum Aufnehmen eines Bilds eines Objektfelds an einem menschlichen oder tierischen Körper von einer Position außerhalb des menschlichen bzw. tierischen Körpers umfasst einen Schaft, der vorzugsweise lang erstreckt und starr ausgebildet ist, und eine an einem distalen Ende des Schafts angeordnete Beobachtungsoptik zum Aufnehmen eines Bilds des Objektfelds. Alternativ kann der Schaft auch sehr kurz ausgebildet sein. Das Objektfeld ist an einer Oberfläche des menschlichen oder tierischen Körpers angeordnet oder zumindest teilweise von außerhalb des Körpers beobachtbar, insbesondere ist das Objektfeld ein Operationsfeld einer chirurgischen Operation. Außerhalb bedeutet hier, dass die Vorrichtung sich zu jedem Zeitpunkt vollständig außerhalb des Körpers befindet. Vorzugsweise hat die Vorrichtung einen Abstand von mindestens 15 cm zum Körper. Innerhalb des Schafts ist eine Einrichtung zur Weiterleitung des aufgenommenen Bilds zu einem proximalen Endbereich des Schafts aufgenommen, wo Anschlüsse zum Anschließen von Versorgungs-, Auswertungs- und/oder Anzeigeeinrichtungen vorgesehen sein können. Insbesondere kann ein Versorgungs- und/oder Signalkabel eines der Beobachtungsoptik zugeordneten elektronischen Bildaufnehmers vom distalen Ende zum proximalen Endbereich des Schafts in dem Schaft geführt sein. Ferner können im Schaft beispielsweise Lichtleiter zum Weiterleiten von Beleuchtungslicht zum distalen Ende des Schafts angeordnet sein, wo eine Beleuchtungseinrichtung zum Beleuchten des Objektfelds vorgesehen sein kann. Bevorzugterweise ist die Beleuchtungseinrichtung proximal der Beobachtungsoptik im distalen Endbereich der Vorrichtung angeordnet.

Erfindungsgemäß ist die Beobachtungsoptik als Stereooptik ausgebildet mit mindestens einem elektronischen Bildaufnehmer zum Aufnehmen eines Stereobilds des Objektfelds. Der mindestens eine elektronische Bildaufnehmer kann beispielsweise ein CCD (chargecoupled device) sein. Eine solche Stereooptik umfasst insbesondere eine Mehrzahl an Objektiven, die in einer Richtung quer zu einer Blickrichtung der Beobachtungsoptik beabstandet sind, und die jeweils ein Bild des Objektfelds auf jeweils einem oder auch einem gemeinsam zugeordneten elektronischen Bildaufnehmer erzeugen. Die Objektive und Bildaufnehmer sind innerhalb der Optikeinheit angeordnet. Insbesondere sind die Objektive in einer festen räumlichen Beziehung zueinander angeordnet, wobei der quer zur Blickrichtung gemessene Abstand zwischen zwei Objektiven die Stereobasis darstellt. Die Objektive sind mit ihren jeweiligen optischen Achsen parallel oder in einem Winkel zueinander, der durch die Stereobasis und den gewünschten Arbeitsabstand bestimmt ist, angeordnet. Durch eine winklige Anordnung kann es erreicht werden, dass bei dem bevorzugten Arbeitsabstand die Mitte jedes Halbbilds des stereoskopischen Bilds denselben Punkt des Objektfelds darstellt. Als "Blickrichtung" der Beobachtungsoptik wird in dem Fall, dass die optischen Achsen der Objektive in einem Winkel zueinander stehen, eine zwischen den optischen Achsen stehende mittlere Richtung bezeichnet.

Erfindungsgemäß ist die als Stereooptik ausgebildete Beobachtungsoptik von einer Optikeinheit umfasst, die um eine zur Blickrichtung näherungsweise parallele erste Drehachse drehbar ausgebildet ist. Die Optikeinheit ist am distalen Ende des Schafts angeordnet, beispielsweise in einem distalen Endabschnitt des Schafts oder in einem am distalen Endabschnitt des Schafts angeordneten Kopfteil der Vorrichtung. Vorzugsweise ist die erste Drehachse parallel zur Blickrichtung der Beobachtungsoptik gerichtet oder nur um maximal einen solchen Winkel gegenüber der Blickrichtung geneigt, dass derjenige Punkt, in dem die erste Drehachse das Objektfeld durchstößt, in den stereoskopischen Halbbildern unabhängig von der Drehung der Optikeinheit um die erste Drehachse enthalten ist. Die Stereooptik kann selbst die drehbare Optikeinheit bilden. Vorzugsweise ist die Optikeinheit als kompakte Einheit ausgebildet, in der die Stereooptik aufgenommen ist, und weist insbesondere eine zylindrische Mantelfläche auf. Die Optikeinheit kann weitere mechanische, optische und/oder elektronische Bauelemente umfassen.

Dadurch, dass die Vorrichtung eine um eine zur Blickrichtung näherungsweise parallele erste Drehachse drehbare Optikeinheit aufweist, die eine als Stereooptik ausgebildete Beobachtungsoptik umfasst, wird es ermöglicht, ein stereoskopisches Bild des Objektfelds aufzunehmen und bei einem Schwenken der erfindungsgemäßen Vorrichtung sowohl eine Aufrichtung des aufgenommenen und dargestellten Bilds des Objektfelds als auch eine Einstellung der Stereobasis vorzunehmen. Hierdurch wird es ermöglicht, auch nach einem Schwenken der Vorrichtung das aufgenommene stereoskopische Bild mit einer im Wesentlichen ungeänderten Ausrichtung sowie in einer solchen Weise darzustellen, dass für einen Benutzer bzw. Beobachter der Stereoeffekt wahrnehmbar ist. Ein Verlorengehen des räumlichen Eindrucks und eine Erschwerung der Orientierung durch Rotation der dargestellten Bilder und der Stereobasis auf einem Bildschirm kann dadurch vermieden werden.

Vorzugsweise umfasst die Optikeinheit mindestens zwei elektronische Bildaufnehmer, von denen jeder einem Objektiv zur Aufnahme eines Halbbilds des stereoskopischen Bilds des Objektfelds zugeordnet ist. In besonders bevorzugter Weise umfasst die Optikeinheit genau zwei Objektive und zwei elektronische Bildaufnehmer, die jeweils ein Halbbild des stereoskopischen Bilds aufnehmen. Innerhalb der Optikeinheit sind die beiden Objektive mit ihren jeweils zugeordneten Bildaufnehmern fest angeordnet, wobei der quer zur Blickrichtung gemessene Abstand die Stereobasis und die Winkelhalbierende zwischen den optischen Achsen der Objektive die Blickrichtung darstellt. Dadurch, dass die Optikeinheit zwei fest in der Optikeinheit angeordnete Objektive mit jeweils einem elektronischen Bildaufnehmer umfasst, wird eine besonders einfache Anordnung zur Aufnahme eines stereoskopischen Bilds ermöglicht.

Die Optikeinheit kann ferner weitere Bildaufnehmer zur Aufnahme von Bildern, insbesondere in bestimmten Wellenlängenbereichen aufweisen. Beispielsweise können Bildsensoren oder optische Bauteile für die Fluoreszenzdiagnostik vorgesehen sein. Die Bildsensoren können ausgebildet sein, Licht in nicht sichtbaren Wellenlängenbereichen zu erfassen.

Gemäß einer bevorzugten Ausführungsform der Erfindung weist die Vorrichtung ein hermetisch dichtes Gehäuse auf, innerhalb dessen die Optikeinheit aufgenommen und drehbar gelagert ist. Insbesondere ist das Gehäuse derart hermetisch dicht ausgebildet, dass auch unter den bei der Sterilisation in einem Autoklaven auftretenden Temperatur- und Druckbedingungen kein Eindringen von Dampf in das Gehäuse möglich ist oder zumindest nicht in einem wesentlichen Umfang erfolgen kann. Das Gehäuse bildet somit eine Hülle, durch die die Optikeinheit hermetisch von der Umgebung getrennt ist. Vorzugsweise ist das Gehäuse metallisch, insbesondere aus einem nicht-ferromagnetischen Metall, ausgebildet; hierfür sind beispielsweise Aluminium und austenitische Stähle geeignet. Das Gehäuse weist mindestens ein transparentes, hermetisch dicht eingesetztes Fenster auf, durch das die Stereooptik die jeweiligen Halbbilder, die das Objektfeld abbilden, aufnimmt. Das Gehäuse, das vorzugsweise relativ zur Vorrichtung feststehend ist, kann beispielsweise als Gehäuse eines am distalen Ende des Schafts angeordneten Kopfteils ausgebildet sein, das gegenüber dem Schaft erweitert ist, kann aber auch selbst einen distalen Endabschnitt des Schafts darstellen. Das Gehäuse kann auf einen distalen Endbereich der Vorrichtung beschränkt sein oder sich in den Schaft hinein erstrecken bzw. zusammen mit dem Schaft eine hermetisch dichte Hülle bilden. Es kann auch die gesamte Vorrichtung umfassen, so dass alle Bauteile in dem Gehäuse nach außen hermetisch dicht abgeschlossen angeordnet sind. Dadurch, dass die Vorrichtung ein hermetisch dichtes Gehäuse aufweist, innerhalb dessen die Optikeinheit drehbar aufgenommen ist, wird es ermöglicht, die Vorrichtung als autoklavierbares Instrument auszubilden. Hierdurch können auf einfache Weise die hygienischen Anforderungen bei einem Einsatz im sterilen Bereich eines Operationssaals erfüllt werden, ohne dass die Lebensdauer der in der Optikeinheit enthaltenen optischen und elektronischen Bauelemente wesentlich eingeschränkt würde.

Bevorzugterweise weist eine Beleuchtungseinrichtung am distalen Ende der Vorrichtung ein Fenster auf, das hermetisch dicht mit dem Gehäuse verbunden ist.

Zur Drehung der Optikeinheit zur Aufrichtung des aufgenommenen Bilds und zur Einstellung der Stereobasis kann innerhalb des hermetisch dichten Gehäuses ein elektromotorischer Antrieb vorgesehen sein, der von außerhalb des Gehäuses angesteuert werden kann. Insbesondere in dem Fall, dass eine Schwenkbewegung der Vorrichtung durch einen oder mehrere Sensoren erfasst wird, kann hierdurch eine automatische Ausrichtung ermöglicht werden. Eine derartige Schwenkbewegung kann beispielsweise durch Schwerkraftsensoren, Inertialsensoren oder Sensoren eines Ortungssystems erfassbar sein.

Gemäß einer bevorzugten Ausführungsform der Erfindung ist zum Bewerkstelligen der Drehung der Optikeinheit ein Magnettrieb vorgesehen, der mindestens ein außerhalb des Gehäuses angeordnetes erstes magnetisches Koppelelement umfasst, das mit mindestens einem innerhalb des Gehäuses angeordneten zweiten magnetischen Koppelelement zusammenwirkt. Das zweite magnetische Koppelelement ist der Optikeinheit zum Drehen der Optikeinheit um die erste Drehachse zugeordnet. Als magnetische Koppelelemente können insbesondere Permanentmagneten vorgesehen sein, wobei die ersten und die zweiten magnetischen Koppelelemente einander mit gleichen oder ungleichen Polen gegenüber stehend angeordnet sein können, um durch magnetische Anziehungs- bzw. Abstoßungskräfte eine magnetische Kopplung zu bewirken. Als magnetische Koppelelemente können auch nicht magnetisierte ferromagnetische Elemente dienen, die mit permanentmagnetischen Koppelementen durch die erzeugten Anziehungskräfte zusammenwirken. Das bzw. die ersten magnetischen Koppelelemente können auf einem ersten Magnetträger angeordnet sein, der von außen betätigbar ist, und das bzw. die zweiten magnetischen Koppelelemente auf einem zweiten Magnetträger, der mit der Optikeinheit drehfest verbunden ist. Zwischen den ersten und den zweiten Koppelelementen ist mindestens eine Wandung des Gehäuses angeordnet, durch die hindurch die magnetische Kopplung wirkt, und die zu diesem Zweck ausreichend dünnwandig und/oder aus einem nicht-ferromagnetischen Material ausgebildet ist. Der Magnettrieb kann manuell oder motorisch von außerhalb des Gehäuses betätigbar sein, um die Optikeinheit um die erste Drehachse zu drehen. Auf diese Weise wird eine Drehung der Optikeinheit durch eine Betätigung von außerhalb des hermetisch dichten Gehäuses ermöglicht. Hierdurch kann ein autoklavierbares Exoskop geschaffen werden, bei dem eine Aufrichtung des aufgenommenen Bilds sowie eine Ausrichtung der Stereobasis auf besonders einfache Weise möglich ist.

Weiterhin ist es bevorzugt, dass der Magnettrieb mit einem Bedienelement, etwa einem Drehrad, drehfest verbunden ist, das um eine zur ersten Drehachse im Wesentlichen parallele und vorzugsweise mit dieser zusammenfallende zweite Drehachse drehbar ist. Hierdurch wird auf einfache und intuitive Weise eine manuelle Verstellung der Optikeinheit ermöglicht. Ein Benutzer kann somit nach einem Schwenken des Exoskops durch einfaches Drehen des Bedienelements sowohl das wiedergegebene Bild aufrichten als auch den Stereoeffekt wieder herstellen. Das Bedienelement kann beispielsweise als Drehhebel oder als Drehrad ausgebildet sein und zur Erleichterung der Bedienung Griffmulden bzw. Griffrillen aufweisen. Insbesondere kann der erste Magnetträger, der die ersten magnetischen Koppelelemente trägt, mit dem Drehrad drehfest verbunden oder selbst als solches Drehrad ausgebildet sein, wodurch eine besonders einfache Ausgestaltung der Vorrichtung ermöglicht wird.

Gemäß einer besonders bevorzugten Ausführungsform der Erfindung ist die Vorrichtung mit einer relativ zu einer Längsachse des Schafts abgewinkelten Blickrichtung der Beobachtungsoptik ausgebildet.

Insbesondere ist die Blickrichtung näherungsweise um 90° zur Längsachse des Schafts ausgerichtet.

Für ein Exoskop ist eine abgewinkelte Blickrichtung, insbesondere senkrecht zum Schaft, besonders vorteilhaft, da sie es ermöglicht mit der Vorrichtung eine Stelle zu beobachten und zu beleuchten, ohne dass die Vorrichtung dabei einer Bedienperson im Weg wäre. Vor allem wenn die Person auf die gleiche Stelle blickt und sich dabei hinter dem Instrument befindet, ist es vorteilhaft, wenn dieses mit all seinen proximalen Anschlüssen seitlich von der beobachteten Stelle und von der Person weg führt. Bevorzugterweise ist auch eine vorhandene Beleuchtungseinheit in Blickrichtung ausgerichtet.

In einer besonders bevorzugten Ausgestaltung der Erfindung ist das Bedienelement als Drehkappe ausgebildet, die auf ein Kopfteil des Schafts, das die drehbare Optikeinheit enthält, auf einer der Blickrichtung entgegengesetzten Seite aufgesetzt ist. Das Kopfteil ist insbesondere gegenüber dem Schaft, an dessen distalem Ende es angeordnet ist, erweitert. Das Kopfteil kann selbst als hermetisch dichtes Gehäuse ausgebildet sein, als Teil eines solchen Gehäuses oder kann zumindest einen Teil eines hermetisch dichten Gehäuses umfassen, wobei die Optikeinheit im Inneren des hermetisch dichten Gehäuses drehbar gelagert ist. Dadurch, dass das Bedienelement als auf eine Seite des Kopfteils aufgesetzte Drehkappe ausgebildet ist, wird eine einfache Montage der Vorrichtung sowie eine einfache und intuitive Verstellung der Optikeinheit ermöglicht.

Vorzugsweise ist das mindestens eine zweite magnetische Koppelelement an einer äußeren Mantelfläche des zweiten Magnetträgers angeordnet, der im Wesentlichen zylindrisch ausgebildet ist und der mit der Optikeinheit drehfest und in dem hermetisch dichten Gehäuse aufgenommen ist. Insbesondere ist eine Mehrzahl von zweiten magnetischen Koppelelementen vorhanden, die an der äußeren Mantelfläche des zweiten Magnetträgers in Umfangsrichtung gleichmäßig verteilt sein können. Das mindestens eine erste magnetische Koppelelement ist dementsprechend an einer Innenseite eines mit dem Bedienelement drehfest verbundenen ersten Magnetträgers angeordnet, der als zylindrischer Drehring ausgebildet und außerhalb des Gehäuses angeordnet ist. Der erste Magnetträger umschließt den zweiten Magnetträger auf dessen Außenseite mit einem geringen Abstand, wobei zwischen dem ersten und dem zweiten Magnetträger eine Wand des Gehäuses angeordnet ist. Vorzugsweise weist der erste Magnetträger eine Mehrzahl von ersten magnetischen Koppelelementen auf, die an der Innenseite des ersten Magnetträgers in Umfangsrichtung gleichmäßig verteilt sein können und die derart angeordnet sind, dass sie mit einer Mehrzahl von zweiten magnetischen Koppelelementen zum Übertragen eines Drehmoments vom ersten auf den zweiten Magnetträger zusammenwirken. Dadurch, dass das bzw. die der Optikeinheit zugeordneten zweiten magnetischen Koppelelemente an der äußeren Mantelfläche des zweiten Magnetträgers und das bzw. die dem Bedienelement zugeordneten ersten magnetischen Koppelelemente auf der inneren Oberfläche des ersten Magnetträgers angeordnet sind, wobei der erste den zweiten Magnetträger umgreift, wird eine magnetische Kopplung mit einem besonders großen Durchmesser und somit die Übertragung eines besonders großen Drehmoments sowie eine besonders feinfühlige Verstellung der Optikeinheit ermöglicht.

Gemäß einer weiteren bevorzugten Ausführungsform ist der erste Magnetträger, der das mit dem Bedienelement verbundene mindestens eine erste magnetische Koppelelement trägt, im Wesentlichen zylindrisch ausgebildet, wobei das mindestens eine erste magnetische Koppelelement an einer Mantelfläche des ersten Magnetträgers angeordnet ist. Dementsprechend ist der zweite Magnetträger, der das mit der Optikeinheit verbundene mindestens eine zweite magnetische Koppelelement trägt, im Wesentlichen ringförmig ausgebildet, wobei das mindestens eine zweite magnetische Koppelelement an einer Innenseite des zweiten Magnetträgers angeordnet ist. Der erste Magnetträger greift in den zweiten Magnetträger ein, wobei zwischen dem ersten und dem zweiten Magnetträger ein geringer Abstand verbleibt, in dem eine Wand des Gehäuses angeordnet ist. Das Gehäuse ist in diesem Bereich somit topfförmig ausgebildet und umschließt den ringförmigen zweiten Magnetträger innen- und außenseitig, wobei der erste Magnetträger in die durch die topfförmige Ausgestaltung gebildete Einsenkung eingreift. Hierdurch wird eine besonders sichere Verbindung zwischen dem ersten Magnetträger und dem Gehäuse ermöglicht, insbesondere in dem Fall, dass außerhalb des zweiten Magnetträgers ein diesen und eine weitere Wand des Gehäuses übergreifendes Drehrad angeordnet ist.

In vorteilhafter Weise kann es auch vorgesehen sein, dass erste magnetische Koppelelemente sowohl an einer Innenseite eines mit dem Bedienelement drehfest verbundenen Drehrings als auch an der Außenseite eines mit dem Bedienelement drehfest verbundenen Zylinders angeordnet sind, wobei zwischen dem Zylinder und dem Drehring ein Spalt gebildet ist. Die zweiten magnetischen Koppelelemente sind in diesem Fall sowohl an einer äußeren Mantelfläche eines mit der Optikeinheit drehfest verbundenen zylindrischen Rings, der in den Spalt zwischen dem Zylinder und dem Drehring eingreift, als auch an einer Innenseite des zylindrischen Rings angeordnet oder sind derart in dem zylindrischen Ring aufgenommen, dass eine magnetische Kopplungswirkung sowohl zu den innenliegenden als auch zu den außenliegenden zweiten magnetischen Koppelelementen ermöglicht wird. Der mit der Optikeinheit drehfest verbundene Ring ist innen- und außenseitig von Wänden des Gehäuses umschlossen. Hierdurch werden eine besonders wirksame Kopplung und eine besonders feinfühlige Verstellung der Optikeinheit ermöglicht.

Vorzugsweise sind sowohl mindestens ein Anschlag für die Drehbewegung der Optikeinheit als auch mindestens ein Anschlag für die Drehung des Bedienelements vorgesehen, die zur Begrenzung des jeweils möglichen maximalen Umdrehungswinkels dienen. In der Regel ist ein maximaler Umdrehungswinkel von ± 90° vorteilhaft und ausreichend. Ferner wird es hierdurch ermöglicht, durch Verstellung gegen den Anschlag des Bedienelements eine feste Winkelbeziehung zwischen dem Bedienelement und der Optikeinheit zu erhalten. Insbesondere kann dadurch ein unerwünschtes Überspringen einzelner Koppelelemente rückgängig gemacht werden, beispielsweise indem am Bedienelement weiter ein Moment aufgebracht wird, nachdem die Optikeinheit ihren Anschlag erreicht hat. Sobald beide Anschläge erreicht sind, ist die ursprüngliche Kopplung des Bedienelements mit der Optikeinheit wieder hergestellt. Hierdurch wird die Bedienung erleichtert.

Weiterhin ist es bevorzugt, dass das Bedienelement eine fühlbare Rastung aufweist, so dass eine oder mehrere bevorzugte Winkelstellungen der Optikeinheit für einen Bediener leicht erkennbar sind. Eine bevorzugte Stellung der Optikeinheit kann beispielsweise eine solche sein, bei der eine Längsmittelebene zwischen den optischen Achsen der beiden Objektive senkrecht zur Längsachse des Schafts steht oder eine solche, in der die Längsachse des Schafts in der Mittelebene zwischen den beiden optischen Achsen liegt. Ferner kann eine fühlbare oder sichtbare, etwa farblich hervorgehobene Markierung an dem Bedienelement vorgesehen sein, um eine bevorzugte Winkelstellung erkennbar zu machen. Hierdurch wird die Bedienung der Vorrichtung weiter vereinfacht.

Vorzugsweise ist die Optikeinheit wärmeleitend mit einem im Schaft verlaufenden Wärmeleiter verbunden. Hierdurch wird die Abführung der beim Betrieb des bzw. der elektronischen Bildaufnehmer entstehenden Verlustwärme ermöglicht, so dass eine Erwärmung einer äußeren Oberfläche des Exoskops auf eine unzulässige Temperatur, insbesondere auf eine Temperatur oberhalb von ca. 48°C, vermieden werden kann. Hierfür kann es beispielsweise vorgesehen sein, dass eine im Schaft verlaufende Heatpipe mit ihrem distalen Ende in eine Nut eines der Optikeinheit benachbarten Bereichs des Gehäuses eingreift, das die Optikeinheit umschließt, so dass nur ein geringer Spalt zwischen der Optikeinheit und dem an die Heatpipe angekoppelten Bereich des Gehäuses besteht, oder direkt an einer Außenseite der Optikeinheit anliegt. Alternativ oder zusätzlich kann die Optikeinheit auf wärmeleitenden Gleitlagern, beispielsweise aus Bronze oder Graphitfolie, gelagert sein. Weiter alternativ oder zusätzlich kann ein Abstreifer aus einem metallischen Material oder auch aus Graphit an einer Außenfläche der Optikeinheit zur Wärmeabführung anliegen. Die auf diese Weise in den Schaft übertragene Wärme kann ebenfalls durch eine Heatpipe zum proximalen Ende des Schafts abführbar sein, wo sie in den Griff weitergeleitet und über die Oberfläche des Griffs abgegeben werden kann; im proximalen Endbereich des Schafts oder im Griff kann aber auch ein Wärmetauscher zur weiteren Abführung der Wärme in eine externe Kühleinrichtung angeordnet sein. Durch derartige Maßnahmen zur Wärmeabführung kann auch die Abführung von durch die Beleuchtungseinrichtung in den Schaft und dessen distalen Endbereich eingebrachter Verlustwärme ermöglicht werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b ein Ausführungsbeispiel einer erfindungsgemäßen Vorrichtung in zwei unterschiedlichen perspektivischen Ansichten;
Fig. 2a bis 2c ein erstes Ausführungsbeispiel einer erfindungsgemäßen Vorrichtung in einer Seitenansicht und zwei Schnittansichten;
Fig. 3 eine weitere Schnittansicht des ersten Ausführungsbeispiels;
Fig. 4 eine Schnittansicht eines zweiten Ausführungsbeispiels einer erfindungsgemäßen Vorrichtung;
Fig. 5 eine Schnittansicht eines dritten Ausführungsbeispiels einer erfindungsgemäßen Vorrichtung.

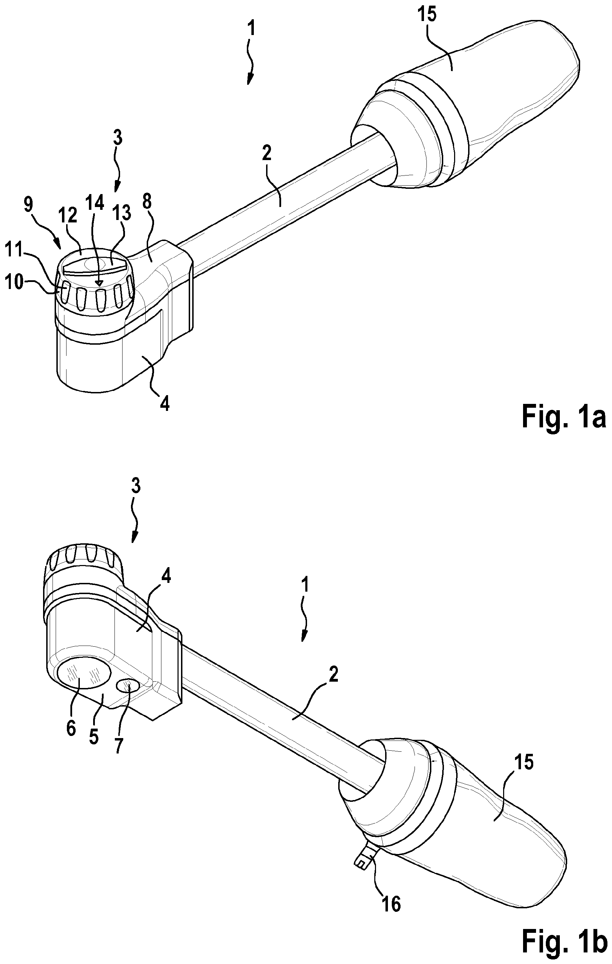

Eine erfindungsgemäße Vorrichtung, die im Folgenden als Exoskop 1 bezeichnet wird, ist in den Figuren 1a und 1b in zwei unterschiedlichen perspektivischen Ansichten gezeigt. Das Exoskop 1 weist einen lang erstreckten zylindrischen Schaft 2 auf, an dessen distalem Ende ein im Vergleich zum Schaft erweitertes Kopfteil 3 angeordnet ist. Das Kopfteil 3 weist ein Gehäuse 4 auf, dessen distaler Bereich näherungsweise halbzylindrisch geformt ist, wobei die Achse des Halbzylinders zur Längsachse des Schafts 2 in einem Winkel von etwa 90° steht. In eine Bodenfläche 5 des Gehäuses ist ein Beobachtungsfenster 6 eingesetzt, durch das eine im Inneren des Gehäuses 4 angeordnete Beobachtungsoptik Licht von einem Objektfeld erhält. Die Bodenfläche 5 weist ferner ein Beleuchtungsfenster 7 auf, hinter bzw. in dem eine Beleuchtungsoptik endet und durch das Beleuchtungslicht zum Beleuchten des Objektfelds abgestrahlt werden kann. Die Beleuchtungsoptik kann beispielsweise eine hinter dem Beleuchtungsfenster 7 im Inneren des Kopfteils angeordnete Lichtquelle sein. Es können aber auch Lichtleiter im Bereich des Beleuchtungsfensters 7 enden, oder es können die Endflächen der Lichtleiter selbst das Beleuchtungsfenster 7 bilden, wobei die Lichtleiter durch den Schaft 2 geführt sind und Licht von einer außerhalb des Kopfteils 3 angeordneten Lichtquelle übertragen. Das Beobachtungsfenster 6 ist vorzugsweise eine planparallele Platte, während das Beleuchtungsfenster vorzugsweise als Konvexlinse ausgebildet ist.

Auf einen distalen Endbereich einer der Bodenfläche 5 gegenüberliegenden Deckelfläche 8 ist eine Drehkappe 9 aufgesetzt, die an ihrer Umfangsseite 10 eine Mehrzahl von Griffmulden 11 aufweist und auf ihrer Oberseite 12 einen Drehgriff 13 trägt. Ferner weist die Drehkappe 9 eine Markierung 14 auf, die die Drehstellung der Drehkappe 9 leicht erkennbar macht. Das Exoskop 1 weist ferner einen Handgriff 15 auf, der einen Anschluss 16 zum Anschließen von Signal- und Versorgungskabeln aufweist. Der Handgriff 15 kann auch beispielsweise eine Kühleinrichtung oder eine Lichtquelle zur Erzeugung von Beleuchtungslicht, das durch Lichtleiter zum Beleuchtungsfenster 7 geleitet wird, enthalten.

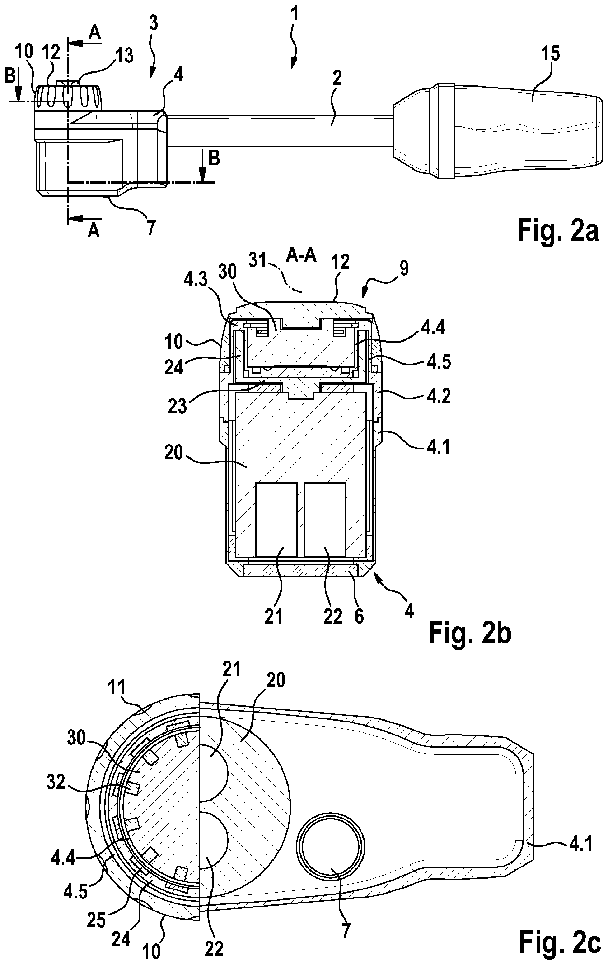

Die im Folgenden beschriebenen und in den Figuren 2a bis 5 dargestellten Ausführungsformen einer erfindungsgemäßen Vorrichtung entsprechen in ihrer äußeren Ansicht dem in Fig. 1a und 1b dargestellten Exoskop 1.

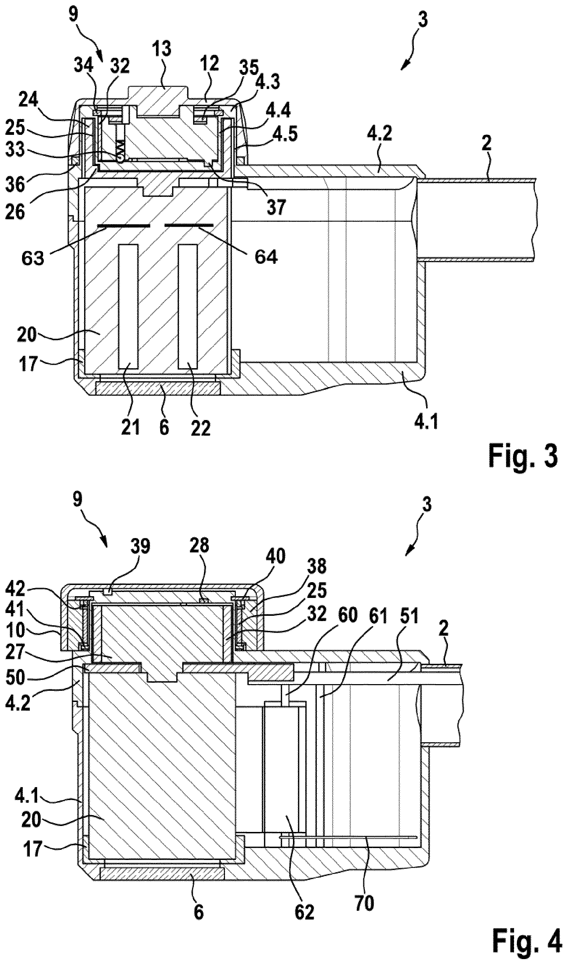

Ein Exoskop gemäß einer ersten Ausführungsform der Erfindung ist in den Figuren 2a bis 2c sowie in Fig. 3 dargestellt. Die Fig. 2a zeigt eine Seitenansicht eines solchen Exoskops 1, die dem in den Figuren 1a und 1b gezeigten entspricht; insofern wird auf die Beschreibung zu Fig. 1a und 1b verwiesen. In Fig. 2a sind zwei Schnittlinien gezeigt, wobei der Schnitt A-A bezogen auf die Längsachse des Schafts 2 einen Querschnitt und der Schnitt B-B einen waagrechten Längsschnitt in zwei unterschiedlichen Ebenen darstellt.

Wie in der Querschnittsdarstellung der Fig. 2b gezeigt ist, ist das Gehäuse 4 aus einem Gehäuseunterteil 4.1 und einem Gehäuseoberteil 4.2 zusammengesetzt, die hermetisch dicht, beispielsweise durch Schweißen, miteinander verbunden sind. Das Beobachtungsfenster 6 ist hermetisch dicht in die von dem Gehäuseunterteil 4.1 gebildete Bodenfläche 5 eingesetzt, etwa durch Löten, ebenso wie das in Fig. 2c erkennbare Beleuchtungsfenster 7. Das Gehäuse 4 ist somit insgesamt hermetisch dicht. Im Inneren des Gehäuses 4 ist eine Optikeinheit 20 angeordnet, die zwei symbolisch dargestellte Objektive 21, 22 sowie weitere, nicht dargestellte Bauelemente, insbesondere zwei den Objektiven 21, 22 zugeordnete elektronische Bildaufnehmer sowie ggf. eine Vorauswertungselektronik, enthält. Die Blickrichtung der Optikeinheit bzw. der Objektive ist in einem Winkel von etwa 90° zur Längsachse des Schafts 2 ausgerichtet.

Mit der Optikeinheit 20 drehfest verbunden ist eine Scheibe 23, die an ihrem äußeren Umfang einen hochstehenden hohlzylindrischen zweiten Magnetträger 24 trägt. Die Scheibe 23 und der zweite Magnetträger 24 sind ebenfalls vollständig vom hermetisch dichten Gehäuse 4 umschlossen, wofür dieses einen hohlen, hochstehenden Ringbereich aufweist, der durch eine Innenwand 4.4, eine Außenwand 4.5 und einen Deckbereich 4.3 gebildet wird. Das Gehäuseoberteil 4.2 weist in seinem oberen Bereich dadurch eine topfförmige Einsenkung auf.

Auf das Gehäuseoberteil 4.2 ist die Drehkappe 9 drehbar aufgesetzt, deren Umfangsbereich 10 die Außenwand 4.5 des Gehäuseoberteils 4.2, die den ringförmigen zweiten Magnetträger 24 umschließt, außenseitig umgreift. Der Umfangsbereich 10 der Drehkappe 9 oder die Drehkappe 9 insgesamt ist aus einem geeigneten, gering wärmeleitfähigen Kunststoffmaterial ausgebildet, um für einen Benutzer auch dann, wenn das Kopfteil 3 durch die innerhalb desselben freigesetzte Verlustwärme erwärmt ist, ein angenehmes Betätigen der Drehkappe 9 zu ermöglichen. Mit der Drehkappe 9 ist ein zylindrischer erster Magnetträger 30 drehfest verbunden, der in die topfförmige Einsenkung des Gehäuseoberteils 4.2 mit einem geringen Spiel eingesetzt ist. Die Drehkappe 9 mit dem Zylinder 30 ist am Gehäuse 4 drehbar gelagert. Die Optikeinheit 20 mit der Scheibe 23 und dem zweiten Magnetträger 24 ist innerhalb des Gehäuses 4 drehbar gelagert, wobei die Optikeinheit 20 und die Drehkappe 9 eine gemeinsame Drehachse 31 aufweisen. Die Drehachse 31 stellt die Mittelachse der im Wesentlichen zylindrisch geformten Optikeinheit 20, der Scheibe 23, der Magnetträger 24, 30 und der Drehkappe 9 dar. Die Objektive 21, 22 sind innerhalb der Optikeinheit 20 symmetrisch zur Drehachse 31 angeordnet.

Wie in Fig. 2c in einem waagrechten Längsschnitt dargestellt, ist in Ausnehmungen der Mantelfläche des zylindrischen Magnetträgers 30 eine Mehrzahl von Magneten 32 eingesetzt, die gleichmäßig entlang des Umfangs verteilt sind. In Ausnehmungen der inneren Zylinderfläche des hohlzylindrischen zweiten Magnetträgers 24 ist die gleiche Anzahl von Magneten 25 eingesetzt. Die einander gegenüber liegenden Magneten 25, 32 sind jeweils gegenpolig ausgerichtet, so dass bei einem ersten Magnet 25 der magnetische Nordpol nach oben und bei einem diesem gegenüberliegend angeordneten zweiten Magnet 32 der Südpol nach oben gerichtet ist oder umgekehrt. Es kann auch vorgesehen sein, dass bei dem ersten Magnet 25 ein magnetischer Pol nach innen, also zum zweiten Magnet 32 hin, gerichtet ist, und bei dem zweiten Magnet 32 der entgegengesetzte Pol nach außen, also zum ersten Magnet 25. Die entlang des Umfangs angeordneten Paare von einander gegenüberliegenden ersten und zweiten Magneten 25, 32 können jeweils gleiche oder alternierende Polungen aufweisen. Durch die auf diese Weise erzeugten magnetischen Anziehungskräfte zwischen den ersten und den zweiten Magneten 25, 32 sind die beiden Magnetträger 24, 30 magnetisch miteinander gekoppelt, so dass bei einer Drehung des mit der Drehkappe 9 verbundenen zweiten Magnetträgers 24 der mit der Optikeinheit 20 verbundene erste Magnetträger 30 folgt, ggf. mit einem von der Reibung der Lagerung der Optikeinheit 20 und der Stärke der magnetischen Kopplung abhängigen Winkelversatz.

Weitere Einzelheiten der beschriebenen ersten Ausführungsform des Exoskops 1 sind in dem in Fig. 3 dargestellten senkrechten Längsschnitt gezeigt. Das Gehäuseunterteil 4.1 und das Gehäuseoberteil 4.2 sind im proximalen Bereich des Kopfteils 3 abgeschlossen oder hermetisch dicht mit dem Schaft 2 verbunden, der in seinem proximalen Endbereich hermetisch dicht abgeschlossen sein kann (nicht dargestellt). Die Optikeinheit 20 enthält zwei in Fig. 3 nur symbolisch angedeutete Objektive 21, 22 sowie die diesen zugeordneten elektronischen Bildaufnehmer 63 und 64. Der über die Scheibe 23 drehfest mit der Optikeinheit 20 verbundene ringförmige zweite Magnetträger 24 trägt an seiner Innenseite eine Mehrzahl von zweiten Magneten 25. Der zylindrische erste Magnetträger 30, der mit der Drehkappe 9 drehfest verbunden ist, trägt an seiner Außenseite eine Mehrzahl von ersten Magneten 32. Die Magneten 25, 32 wirken als magnetische Koppelelemente, die durch die relativ dünne Innenwand 4.4 des Gehäuses 4 hindurch zusammenwirken. Wie oben beschrieben, können hierfür beispielsweise die der Optikeinheit zugeordneten Magneten 25 entlang der Innenwand des zweiten Magnetträgers 24 mit gleicher oder abwechselnder Polung angeordnet sein und auf der Außenwand des Magnetträgers 30 die Magneten 32 in gleicher Anzahl und gleicher oder gegengleicher Anordnung befestigt sein. Hierdurch entsteht eine magnetische Kopplung zwischen den Magnetträgern 24, 30 und damit zwischen der Drehkappe 9 und der Optikeinheit 20, so dass durch Drehen der Drehkappe 9 um die Drehachse 31 (s. Fig. 2b) eine Drehung der Optikeinheit um die gleiche Drehachse bewirkt werden kann.

In dem ersten Magnetträger 30 kann ein Formgehemme in Form einer gefedert gelagerten Rastkugel 33 angeordnet sein, wobei die Rastkugel 33 in entsprechende Ausnehmungen des Gehäuseoberteils 4.2 eingreift. Die Drehkappe 9 ist gemeinsam mit dem ersten Magnetträger 30 durch einen Sprengring 34 an dem ringförmigen Deckbereich 4.3 des Gehäuseoberteils gesichert und mit Gleitdichtringen 35, 36 abgedichtet. Weiterhin ist ein Anschlag 26 zur Begrenzung des Umdrehungswinkels der Optikeinheit 20 an der Scheibe 23 vorgesehen sowie ein Anschlag 37 am Magnetträger 30 zur Begrenzung des Verstellwinkels der Drehkappe 9. Sowohl der Anschlag 26 als auch der Anschlag 37 wirken mit entsprechenden Anschlägen des Gehäuses zusammen und ermöglichen neben der Begrenzung der Winkel auch die Sicherstellung einer optimalen Kopplung zwischen den magnetischen Koppelelementen. Als Verstellwinkelbereich ist in der Regel ein Bereich von ± 90° ausreichend. Die Optikeinheit 20 ist im Gehäuseunterteil 4.1 mit einem Gleitlager 17 gelagert. Im Inneren des Gehäuses 4 ist eine nicht dargestellte Kabelführung zur Führung von ebenfalls nicht dargestellten Versorgungs- und Signalkabeln, die durch den Schaft 2 verlaufen, zur drehbaren Optikeinheit 20 angeordnet.

In Fig. 4 ist der distale Endbereich einer zweiten Ausführungsform eines erfindungsgemäßen Exoskops dargestellt. Dabei ist mit der Optikeinheit 20 ein zylindrischer zweiter Magnetträger 27 drehfest verbunden, der umfangsseitig zweite Magneten 32 trägt, die durch eine Wand des Gehäuseoberteils 4.2 hindurch mit ersten Magneten 25 magnetisch gekoppelt sind, die auf der Innenseite des ringförmigen ersten Magnetträgers 38, der am Umfangsbereich 10 der Drehkappe 9 befestigt ist, angeordnet sind. Der Verstellwinkel der Drehkappe 9 wird durch einen Anschlag 39 begrenzt, während der Umdrehungswinkel der Optikeinheit 20 durch einen Anschlag 28 begrenzt wird. Zur Abdichtung der Drehkappe 9 sind Gleitdichtringe 40, 41 vorgesehen. Im Übrigen kann das zweite Ausführungsbeispiel der Erfindung wie das zuvor beschriebene erste Ausführungsbeispiel ausgebildet sein. Die Objektive 21, 22 und die elektronischen Bildaufnehmer sind in Fig. 4 nicht dargestellt.

In Fig. 4 sind Maßnahmen zur Kabelführung und zur Wärmeabführung dargestellt, die ebenfalls bei dem zuvor beschriebenen Ausführungsbeispiel eingesetzt werden können. Auf der dem Gleitlager 17 gegenüberliegenden Seite ist die Optikeinheit 20 mit Wärmeleitblechen 50 aus Graphit in Kontakt, durch die in der Optikeinheit 20 erzeugte Verlustwärme in eine Heatpipe 51 weitergeleitet wird, wodurch die Verlustwärme durch den Schaft 2 in den proximalen Endbereich des Exoskops 1 bzw. in den Griff 15 abgeführt wird. Zur Führung von Verbindungsleitungen zur Optikeinheit 20 sind Umlenkungen 60, 61 vorgesehen, über die ein Kabel, das beispielsweise als Flexplatine 62 ausgebildet sein kann, geführt wird. Eine Führung 70 zur Verschiebung der Umlenkung 60 ist in Fig. 4 symbolisch angedeutet.

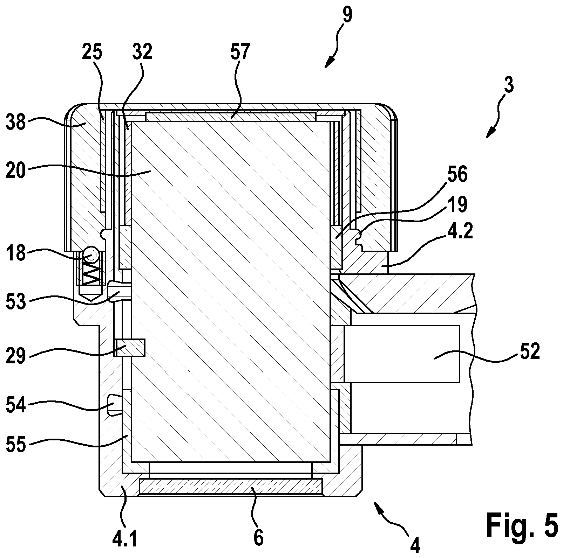

In Fig. 5, die eine dritte Ausführungsform eines erfindungsgemäßen Exoskops zeigt, sind weitere Maßnahmen zur Wärmeabführung dargestellt, die zusätzlich oder alternativ zu den zuvor beschriebenen bei den oben beschriebenen Ausführungsbeispielen zum Einsatz kommen können. Wie in Fig. 5 schematisch dargestellt, kann zur Wärmeableitung eine als Abstreifer 52 ausgebildete Graphitfolie dienen, die an der Mantelfläche der Optikeinheit 20 anliegt. Alternativ oder zusätzlich können Heatpipes 53, 54 form- oder stoffschlüssig in Nuten des Gehäuses 4 eingesetzt sein, um eine effektive Kühlung des Gehäuses 4 und damit der Optikeinheit 20 zu gewährleisten. In Fig. 5 ist ein eine Rastwirkung erzeugendes Formgehemme mit einer gefederten Rastkugel 18 gezeigt, die in unterseitige Ausnehmungen des ersten Magnetträgers 38 eingreift. Ferner sind bei der in Fig. 5 gezeigten Ausführungsform ein Anschlag 19 zur Begrenzung des Verstellwinkels der Drehkappe 9 und ein Anschlag 29 zur Begrenzung des Umdrehungswinkels der Optikeinheit 20 vorgesehen. Das Gehäuseoberteil 4.2 des hermetisch dichten Gehäuses ist bei dieser Ausführungsform mehrteilig ausgeführt, wobei die mehreren Einzelteile des Gehäuseoberteils 4.2, wie auch das Gehäuseoberteil 4.2 und das Gehäuseunterteil 4.1 stoffschlüssig miteinander verbunden sind, beispielsweise durch Schweißen. Die Optikeinheit 20 ist hier über Gleitlager 55, 56 und 57 im Gehäuse gelagert. Im Übrigen entspricht die in Fig. 5 gezeigte Ausführungsform der in Fig. 4 dargestellten.

Zur Beobachtung eines Objektfelds, beispielsweise eines Operationsfelds bei einer chirurgischen Operation, wird das Exoskop 1 mit einem in den Figuren nicht dargestellten Haltearm, der mit einem Greifer am Schaft 2 angreift, in eine Position in einem Abstand von beispielsweise 25 bis 70 cm über dem Operationsfeld gebracht, wobei der Schaft 2 im Wesentlichen waagrecht gerichtet ist. Das Exoskop 1 wird dabei derart orientiert, das es aus einer Richtung in den Raum oberhalb des Operationsfelds hineinragt, so dass der Operateur möglichst wenig behindert wird; das Exoskop 1 kann beispielsweise dem Operateur entgegen gerichtet sein. Weiter wird das Exoskop 1 durch Rotation um die Längsachse des Schafts 2 derart eingerichtet, dass die Blickrichtung des Stereoobjektivs nach unten gerichtet ist, so dass das Operationsfeld durch das aus dem Beleuchtungsfenster 7 austretende Beleuchtungslicht beleuchtet und mit der Stereooptik durch das Beobachtungsfenster 6 beobachtet werden kann. In dieser Position und Orientierung wird das Exoskop 1 mit Hilfe des Halters fixiert. Zur Beobachtung des Operationsfelds wird das aufgenommene stereoskopische Bild auf einer geeigneten Anzeigeeinrichtung für den Operateur und ggf. weitere Personen dargestellt sowie für Dokumentationszwecke gespeichert. Sofern hierbei bereits eine Aufrichtung des angezeigten Bilds und/oder eine Einstellung der Stereobasis für eine optimale räumliche Wahrnehmung notwendig ist, kann dies durch manuelles Drehen der Drehkappe 9 erfolgen.

Im Verlauf der chirurgischen Operation kann es erforderlich sein, das Exoskop neu auszurichten. Insbesondere kann es etwa aufgrund eines Positionswechsels des Operateurs notwendig sein, den Haltearm derart einzurichten, dass das Exoskop aus einer anderen Richtung in den Raum oberhalb des Operationsfelds hineinragt; dies entspricht einer Schwenkung des Exoskops um eine zur Blickrichtung parallele Achse bzw. um eine Mittelachse zwischen den optischen Achsen der Objektive 21, 22. Hierbei ändern sich sowohl die Ausrichtung des angezeigten Bilds als auch die Richtung der Stereobasis, so dass die Orientierung auf dem angezeigten Bild erschwert ist und der räumliche Eindruck verloren gehen kann. Durch manuelles Drehen der Drehkappe 9 kann der Operateur oder eine andere Person das Bild aufrichten und die ursprüngliche Richtung der Stereobasis wieder herstellen, so dass die räumliche Wahrnehmung unbeeinträchtigt ist.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste:

- 1: Exoskop
- 2: Schaft
- 3: Kopfteil
- 4: Gehäuse
- 4.1: Gehäuseunterteil
- 4.2: Gehäuseoberteil
- 4.3: Deckbereich
- 4.4: Wand
- 4.5: Wand
- 5: Bodenfläche
- 6: Beobachtungsfenster
- 7: Beleuchtungsfenster
- 8: Deckelfläche
- 9: Drehkappe
- 10: Umfangsseite
- 11: Griffmulde
- 12: Oberseite
- 13: Drehgriff
- 14: Markierung
- 15: Handgriff
- 16: Anschluss
- 17: Gleitlager
- 18: Rastkugel
- 19: Anschlag
- 20: Optikeinheit
- 21: Objektiv
- 22: Objektiv
- 23: Scheibe
- 24: Magnetträger
- 25: Magnet
- 26: Anschlag
- 27: Magnetträger
- 28: Anschlag
- 29: Anschlag
- 30: Magnetträger
- 31: Drehachse
- 32: Magnet
- 33: Rastkugel
- 34: Sprengring
- 35: Gleitdichtring
- 36: Gleitdichtring
- 37: Anschlag
- 38: Magnetträger
- 39: Anschlag
- 40: Wellensicherungsring
- 41: Gleitdichtring
- 42: Gleitdichtring
- 50: Wärmeleitblech
- 51: Heatpipe
- 52: Abstreifer
- 53: Heatpipe
- 54: Heatpipe
- 55: Gleitlager
- 56: Gleitlager
- 57: Gleitlager
- 60: Umlenkung
- 61: Umlenkung
- 62: Flexplatine
- 63: Bildaufnehmer
- 64: Bildaufnehmer
- 70: Führung

## Patentansprüche

1. Exoskop zum Aufnehmen eines Bildes eines Objektfelds an einem menschlichen oder tierischen Körper von außerhalb des Körpers, umfassend einen Schaft (2) und eine an einem distalen Ende des Schafts (2) angeordnete Beobachtungsoptik zum Aufnehmen des Bildes des Objektfelds, wobei die Beobachtungsoptik als Stereooptik mit mindestens einem elektronischen Bildaufnehmer (63, 64) zum Aufnehmen eines Stereobilds des Objektfelds ausgebildet ist und wobei das Exoskop eine Optikeinheit (20) aufweist, die die Beobachtungsoptik umfasst und die um eine zu einer Blickrichtung der Beobachtungsoptik näherungsweise parallele erste Drehachse (31) drehbar ist, **dadurch gekennzeichnet, dass** die Optikeinheit (20) in einem am distalen Endes des Schafts (2) angeordneten Kopfteil (3) angeordnet ist, wobei die Blickrichtung relativ zu einer Längsachse des Schafts (2) abgewinkelt ist, wobei die Blickrichtung senkrecht zur Längsachse des Schafts ausgerichtet ist, und wobei das Exoskop ein hermetisch dichtes Gehäuse (4) aufweist, innerhalb dessen die Optikeinheit (20) aufgenommen und um die erste Drehachse (31) drehbar gelagert ist.

2. Exoskop nach Anspruch 1, **dadurch gekennzeichnet, dass** die Optikeinheit (20) zwei Objektive (21, 22) und zwei elektronische Bildaufnehmer (63, 64) umfasst, wobei jedem Objektiv (21, 22) ein elektronischer Bildaufnehmer zur Aufnahme eines Halbbilds des Stereobilds des Objektfelds zugeordnet ist.

3. Exoskop nach Anspruch 2, **dadurch gekennzeichnet, dass** zum Drehen der Optikeinheit (20) um die erste Drehachse (31) außerhalb des Gehäuses (4) mindestens ein erstes magnetisches Koppelelement angeordnet ist, das mit mindestens einem innerhalb des Gehäuses (4) angeordneten zweiten magnetischen Koppelelement zusammenwirkt, das mit der Optikeinheit (20) verbunden ist.

4. Exoskop nach Anspruch 3, **dadurch gekennzeichnet, dass** das mindestens eine erste magnetische Koppelelement mit einem manuell betätigbaren Bedienelement verbunden ist, das um eine zur ersten Drehachse (31) im Wesentlichen parallele zweite Drehachse drehbar ist.

5. Exoskop nach einem der Ansprüche 4, **dadurch gekennzeichnet, dass** das Bedienelement als eine auf eine der Blickrichtung entgegengesetzte Seite des Kopfteils (3) aufgesetzte Drehkappe (9) ausgebildet ist.

6. Exoskop nach einem der Ansprüche 4 bis 5, **dadurch gekennzeichnet, dass** das mindestens eine zweite magnetische Koppelelement an einer Außenseite eines mit der Optikeinheit (20) drehfest verbundenen, im Wesentlichen zylindrischen zweiten Magnetträgers (27) und das mindestens eine erste magnetische Koppelelement an einer Innenseite eines mit dem Bedienelement drehfest verbundenen, den zweiten Magnetträger (27) umschließenden, im Wesentlichen ringförmigen ersten Magnetträgers (38) angeordnet ist.

7. Exoskop nach einem der Ansprüche 4 bis 5, **dadurch gekennzeichnet, dass** das mindestens eine zweite magnetische Koppelelement an einer Innenseite eines mit der Optikeinheit (20) drehfest verbundenen, im Wesentlichen ringförmigen zweiten Magnetträgers (24) und das mindestens eine erste magnetische Koppelelement an der Außenseite eines in den im Wesentlichen ringförmigen zweiten Magnetträger (24) eingreifenden, mit dem Bedienelement verbundenen, im Wesentlichen zylindrischen ersten Magnetträgers (30) angeordnet ist.

8. Exoskop nach einem der Ansprüche 4 bis 7, **dadurch gekennzeichnet, dass** für das Bedienelement und für die Optikeinheit (20) jeweils ein gegen das Gehäuse (4) wirkender Anschlag (19, 26, 28, 29, 37, 39) zur Begrenzung der Drehbewegung vorgesehen ist.

9. Exoskop nach einem der Ansprüche 4 bis 8, **dadurch gekennzeichnet, dass** das Bedienelement eine oder mehrere Rastpositionen aufweist.

10. Exoskop nach einem der vorhergehenden Ansprüche, **dadurch gekennzeichnet, dass** die Optikeinheit (20) zur Abführung der in der Optikeinheit (20) entstehenden Verlustwärme wärmeleitend mit einem im Schaft (2) angeordneten Wärmeleiter verbunden ist.
